# EUROPEAN PATENT SPECIFICATION

(11) **EP 1 161 522 B1**
(45) Date of publication and mention of the grant of the patent: **26.07.2006**
(21) Application number: 00916131.6
(22) Date of filing: 08.03.2000
(51) Int. Cl.: C12N 5/06, C12N 5/08, C12N 5/20, C12P 21/08, C07K 16/28, C07K 7/08

(54) **METHOD OF ISOLATING CD8+ CELLS, AND RELATED HYBRIDOMA CELLS, ANTIBODIES AND POLYPEPTIDES**
VERFAHREN ZUR ISOLIERUNG DER CD8+-ZELLEN, UND ENTSPRECHENDE HYBRIDOMAZELLEN, ANTIKÖRPER UND POLYPEPTIDE
PROCEDE D'ISOLATION DES CELLULES CD8+ ET CELLULES, ANTICORPS ET POLYPEPTIDES HYBRIDOMES CORRESPONDANTS

(30) Priority: 12.03.1999 US 124253 P
(43) Date of publication of application: 12.12.2001
(73) Proprietor: Ortho-McNeil Pharmaceutical, Inc., Raritan, NJ 08873-1102 (US)
(72) Inventor: LETURCQ, Didier, San Diego, CA 92109 (US)
(74) Representative: Fisher, Adrian John
(86) International application number: PCT/US2000/005898
(87) International publication number: WO 2000/055305

(56) References cited:
- EP-A- 0 699 907
- WO-A-99/54345
- US-A- 5 645 837
- US-A- 6 001 962

## Description

Throughout this application, various publications are cited. The disclosure of these publications is hereby incorporated by reference into this application to describe more fully the state of the art to which this invention pertains.

### Field of the Invention

This invention relates to a positive selection method for isolating CD8* cells using certain CD8-specific antibodies. The isolated CD8⁺ cells have importance as vehicles for combating viral infections and tumors.

### Background of the Invention

In humans, CD8⁺ cells play a vital role in the immune system's ability to defend against potentially harmful foreign entities, such as bacteria and viruses [1]. CD8⁺ cells circulate in the blood and possess on their surface the CD8 protein. When necessary, these cells are converted into cytotoxic cells (i.e. cell-killing cells) which proceed to destroy foreign cells, viruses, and other harmful pathogens present in the subject [2]. Because of CD8⁺ cells' effective role in host defense, they hold great potential in isolated form as therapeutics for treating disorders such as viral infections and malignancies [3].

In the past, purification of human CD8⁺ cells has been achieved by negative selection. Specifically, peripheral blood mononuclear cells ("PBMC's") are incubated with a cocktail of monoclonal antibodies specific for non-CD8 sub-populations. These sub-populations include, for example, B-cells, CD4⁺ cells, NK cells, macrophages and neutrophils, and each contains specific, non-CD8 "markers". The sub-populations are then removed from the resulting antibody cocktail using magnetic beads [4]. This technique has certain major disadvantages. The first is that several monoclonal antibodies are required for removing non-CD8⁺ cells. The second is that the resulting CD8⁺ population suffers from contamination from non-CD8⁺ cells that possess relatively low levels of non-CD8 markers. Finally, when a magnetic separation procedure is used to remove all non-CD8⁺ cells, a large number of magnetic beads are needed.

### Summary of the Invention

This invention provides a method of isolating CD8⁺ cells which comprises the steps of
(a) contacting a sample of isolated peripheral mononuclear blood cells with a first antibody which specifically binds to a peptide consisting essentially of AAEGLDTQRFSG on CD8 molecules present on the surface of CD8⁺ cells but does not activate the CD8⁺ cells once bound thereto, under conditions permitting the formation of a first complex between the CD8⁺ cell and first antibody;
(b) separating from the sample any first antibody not present in the first complex;
(c) contacting the sample with an immobilized second antibody which specifically binds to the first antibody in the first complex, under conditions permitting the formation of an immobilized, second complex between the first complex and the second antibody, thereby immobilizing the CD8⁺ cells present in the sample;
(d) separating the immobilized second complex from the sample;
(e) contacting the immobilized second complex with an agent comprising a peptide comprising AAEGLDTQRFSG, which, under suitable conditions, causes dissociation of the second complex into CD8⁺ cells and an immobilized third complex between the first antibody and second antibody; and
(f) separating the immobilized third complex from the CD8⁺ cells, thereby isolating the CD8⁺ cells.

This invention also provides a hybridoma cell line which produces a monoclonal antibody which specifically binds to a peptide consisting essentially of AAEGLDTQRFSG on CD8 molecules present on the surface of CD8⁺ cells but does not activate the CD8⁺ cells. This invention further provides monoclonal antibodies produced by each of the instant hybridoma cell lines. Finally, this invention provides related polypeptides, isolated CD8⁺ cells and kits.

### Detailed Description of the Invention

The hybridoma cell lines designated 37B1 and 8G6 were deposited pursuant to, and in satisfaction of, the requirements of the Budapest Treaty on the International Recognition of the Deposit of Microorganisms for the Purposes of Patent Procedure with the American Type Culture Collection (ATCC), 10801 University Boulevard, Manassas, Virginia 2010-2209 under ATCC Accession Nos. HB-12441 and HB-12657, respectively.

This invention provides a method of isolating CD8⁺ cells by employing an anti-CD8 antibody, along with certain other reagents. Specifically, this invention provides a method of isolating CD8⁺ cells which comprises the steps of
(a) contacting a sample of isolated peripheral mononuclear blood cells with a first antibody which specifically binds to a peptide consisting essentially of AAEGLDTQRFSG on CD8 molecules present on the surface of CD8⁺ cells but does not activate the CD8⁺ cells once bound thereto, under conditions permitting the formation of a first complex between the CD8⁺ cell and first antibody;
(b) separating from the sample any first antibody not present in the first complex;
(c) contacting the sample with an immobilized second antibody which specifically binds to the first antibody in the first complex, under conditions permitting the formation of an immobilized, second complex between the first complex and the second antibody, thereby immobilizing the CD8⁺ cells present in the sample;
(d) separating from the resulting immobilized second complex from the sample;
(e) contacting the immobilized second complex with an agent comprising a peptide comprising AAEGLDTQRFSG, which under suitable conditions causes dissociation of the second complex into CD8⁺ cells and an immobilized third complex between the first antibody and second antibody; and
(f) separating the immobilized third complex from the CD8⁺ cells, thereby isolating the CD8⁺ cells.

As used herein, a "CD8⁺ cell" means a T-cell having on its surface the CD8 protein. In the preferred embodiment, the CD8⁺ cells are human CD8⁺ cells. The CD8⁺ cells can be from any CD8⁺ cell-possessing species. "Isolating" CD8⁺ cells means obtaining a population of peripheral mononuclear blood cells wherein the ratio of CD8⁺ cells to non-CD8⁺ cells is at least about 7:1. In the preferred embodiment of this invention, this ratio is at least about 9:1.

This invention employs several types of antibodies which specifically bind to given epitopes. More particularly, this invention uses a "first antibody" which specifically binds to a peptide consisting essentially of AAEGLDTQRFSG on CD8 molecules present on the surface of CD8⁺ cells but does not activate the CD8⁺ cells once bound thereto. Here, CD8⁺ cell "activation" means causing CD8⁺ cells to express γ-interferon ("γ-IFN") . This activation can be measured using routine methods such as sandwich ELISA assays, which can be performed using commercially available kits.

Such first antibodies include, but are not limited to, the monoclonal antibodies produced by the hybridoma cell lines 37B1 (ATCC Accession No. HB-12441) and 8G6 (ATCC Accession No. HB-12657). Conditions which permit these antibodies to bind to but not activate CD8* cells are well known in the art. These conditions are described, for example, in a suitable buffer such as Ca2⁺ and Mg2⁺-free Dulbecco's Phosphate Buffer Saline (DPBS) containing 1% Human Serum Albumin (HSA) and 0.2% sodium citrate and gentle mixing by "end over end" rotation on a rotator set at 4 rpm.

As used herein, the term "antibody" includes, but is not limited to, both naturally occurring and non-naturally occurring antibodies. Specifically, the term "antibody" includes polyclonal and monoclonal antibodies, and binding fragments thereof. Furthermore, the term "antibody" includes chimeric antibodies and wholly synthetic antibodies, and fragments thereof. In one embodiment, the antibody is a monoclonal antibody. The monoclonal antibody can be human, or that of another species including, for example, mouse and rabbit. In this invention, an antibody which "specifically" binds to a stated epitope binds to that epitope with a dissociation constant of at least about 10-fold less than the dissociation constant with which it binds to any other epitope. In one embodiment, this dissociation constant ratio is at least about 100. In the preferred embodiment, this dissociation constant ratio is at least about 10³.

The "second antibody" used in the instant method can be any antibody which specifically recognizes an epitope on any portion of the first antibody. In the preferred embodiment, the second antibody specifically recognizes a portion of the constant (Fc) region of the first antibody. Such anti-Fc antibodies are commercially available and include, for example, sheep anti-mouse antibody immobilized on magnetic beads [5].

The agent that causes dissociation of the immobilized second complex into CD8⁺ cells and immobilized antibodies is an agent comprising a peptide comprising AAEGLDTQRFSG. In the preferred embodiment, this agent is the polypeptide designated CD8-3 comprising the sequence AAEGLDTQRFSG. In one embodiment, the immobilized second antibody comprises an antibody operably affixed to a magnetic bead.

This invention also provides a hybridoma cell line which produces a monoclonal antibody which specifically binds to a peptide consisting essentially of AAEGLDTQRFSG on CD8 molecules present on the surface of CD8* cells but does not activate the CD8* cells. In one embodiment, the hybridoma cell line is selected from the cell lines designated 37B1 (ATCC Accession No. HB-12441) and 8G6 (ATCC Accession No. HB-12657). This invention further provides the monoclonal antibodies produced by each of the instant hybridoma cell lines.

This invention further provides a polypeptide useful for generating the instant monoclonal antibody that consists essentially of the amino acid sequence AAEGLDTQRFSG. In the preferred embodiment, the polypeptide is the polypeptide designated CD8-3 and consists essentially of the amino acid sequence AAEGLDTQRFS. The instant polypeptide can optionally comprise one or more additional amino acid residues at the C-terminal or N-terminal end. In the preferred embodiment, the polypeptide consists essentially of the sequence NKPKAAEGLDTQRFSGKRLG.

This invention further provides a population of CD8* cells isolated by the instant method.

Finally, this invention provides a kit for use in isolating CD8⁺ cells which comprises, in separate compartments, (a) an antibody which specifically binds to the sequence AAEGLDTQRFSG on CD8 molecules present on the surface of CD8⁺ cells, but does not activate the CD8⁺ cells once bound thereto; and (b) an agent which causes the dissociation of a CD8⁺ cell-antibody complex wherein the agent is the polypeptide comprising the sequence AAEGLDTQRFSG.

The instant kit can further comprise reagents useful for performing the binding and dissociation steps of the instant method. The components of the instant kit can either be obtained commercially or made according to well known methods in the art. In addition, the components of the instant kit can be in solution or lyophilized as appropriate. In the preferred embodiment, the kit further comprises instructions for use.

The following procedures relating to the instant invention are routine in the art: isolating peripheral mononuclear blood cells from which the CD8⁺ cells are in turn isolated [6]; separating unbound antibodies and cells from a sample containing bound antibodies and/or cells via centrifugation or spinning membrane; and immobilizing antibodies via polystyrene flasks, columns or beads [4,7].

This invention will be better understood by reference to the Experimental Details which follow, but those skilled in the art will readily appreciate that the specific experiments detailed are only illustrative of the invention as described more fully in the claims which follow thereafter.

### Experimental Details

### Rationale

Human CD8⁺ cells can be isolated from preparations of peripheral blood mononuclear cells (PBMC's) by either positive or negative selection. Positive selection results in a highly-purified population of CD8⁺ cells. Negative selection, while resulting in sufficient numbers of CD8⁺ cells, has low levels of contaminating non-CD8 populations remaining after the selection procedure.

The idea was to generate an antibody which has high affinity for CD8⁺ cells, does not activate the cells during the selection process, and is capable of being easily eluted from the cells. An anti-peptide antibody appeared to meet these criteria. However, it was known that anti-peptide antibodies might be of low affinity and may recognize the linear peptide sequence exclusively, preventing reactivity with native antigen.

It was necessary that the anti-CD8 antibody not activate the cells during the selection process, as it would lessen their ability to effectively act as naive responder cells during in vitro stimulation protocols. The use of peptide release to selectively isolate a cell population has been show by Tseng-Law, et al. [8] for CD34⁺ cells.

### Methods

The CD8 alpha chain was examined for hydrophilic sequences and four peptides selected. All were coupled to keyhole limpet hemocyanin (KLH) as carrier and used to immunize mice. A C-terminal amino acid was added to each of the peptides coupled to KLH to make the monoclonal antibodies. Antisera from the mice were evaluated for the ability to recognize both peptide and native CD8 on the surface of T cells. Only one of the four peptides was capable of recognition of both antigenic forms of CD8. Monoclonal antibodies were generated to this peptide and the resulting antibody used to isolate CD8⁺ cells from a PBMC preparation. The antibody was successful in isolating a population of highly-purified CD8⁺ cells (Table 1) which were not activated by the isolation procedure (Table 2).

**Table 1 Purification of CD8⁺ Cells by Positive Selection Analyzed by Flow Cytometry***

| CELL TYPE | PBMC Fluorescence (Range) | POST SELECTION Fluorescence (Range) |
|---|---|---|
| CD8 T cells | 15 (7-24) | 82 (56-95) |
| CD4 T cells | 36 (14-52) | 2 (0.1-10) |
| CD14 Monocytes | 15 (7-26) | 0.8 (0.2-2) |
| CD15 Neutrophils | 12 (8-21) | 0.6 (0.1-3) |
| CD19 B cells | 2 (0.4-7) | 3 (0.5-9) |
| CD56 NK cells | 6 (2-17) | 6 (0.1-20) |

| | | |
|---|---|---|
| * Summary of 10 normal donors | | |

**Table 2 Activation of CD8⁺ Cells Isolated By Negative or Positive Selection (Assessed by IFNγ Production)**

| Cells | | Negative Selection (pg/ml) | Positive Selection (pg/ml) |
|---|---|---|---|
| un-stimulated | | 20 | 20 |
| allo-stimulation | | 1440 | 3600 |

### References

1. Nabholz M. and H.R. MacDonald (1983) Annual Review of Immunology 1:273-306.
2. Riddell S.R. and P.D. Greenberg (1994) Current Topics in Microbiology and Immunology 189:9-34.
3. Riddell S.R. and P.D. Greenberg (1995) Annual Review of Immunology 13:545-586.
4. Horgan K and S. Shaw (1994) Current Protocols in Immunology 2:7.4.1.
5. Lea T, et al. (1988) Journal of Molecular Recognition 1(1):9-18.
6. Kanof, M.E., et al. (1994) Current Protocols in Immunology 2:7.1.1.
7. Kanof M.E. (1994) Current Protocols in Immunology 2:7:3:1.
8. PCT International Publication No. WO 95/34817.

## Claims

1. A method of isolating CD8+ cells which comprises the steps of:
(a) contacting a sample of isolated peripheral mononuclear blood cells with a first antibody which specifically binds to a peptide consisting essentially of AAEGLDTQRFSG (SEQ ID NO:1), on CD8 molecules present on the surface of human CD8+ cells but does not activate the CD8+ cells once bound thereto, under conditions permitting the formation of a first complex between CD8+ cell and first antibody;
(b) separating from the sample any first antibody not present in the first complex;
(c) contacting the sample with an immobilized second antibody which specifically binds to the first antibody in the first complex, under conditions permitting the formation of an immobilized second complex between the first complex and the second antibody, thereby immobilizing the CD8+ cells present in the sample;
(d) separating the immobilized second complex from the sample;
(e) contacting the immobilized second complex with an agent comprising a peptide comprising AAEGLDTQRFSG (SEQ ID NO:1), which under suitable conditions causes dissociation of the second complex into CD8+ cells and an immobilized third complex between the first antibody and second antibody; and
(f) separating the immobilized third complex from the CD8⁺ cells, thereby isolating the CD8+ cells.

2. The method of claim 1, wherein the CD8⁺ cells are human CD8⁺ cells.

3. The method of claim 1, wherein the first antibody is a monoclonal antibody.

4. The method of claim 3, wherein the monoclonal antibody is produced by a hybridoma cell line selected from the group consisting of the cell line designated 37B1 (ATCC Accession No. HB-12441) and the cell line designated 8G6 (ATCC Accession No. HB-12657).

5. The method of claim 1, wherein the immobilized second antibody comprises an antibody operably affixed to a magnetic bead.

6. The method of claim 1, wherein the agent which causes the dissociation of immobilized third complex is the polypeptide designated CD8-3 comprising the amino acid sequence AAEGLDTQRFSG (SEQ ID NO:1).

7. A hybridoma cell line which produces a monoclonal antibody which specifically binds to a peptide consisting essentially of AAEGLDTQRFSG (SEQ ID NO:1) on CD8 molecules present on the surface of CD8⁺ cells.

8. A hybridoma cell line designated 37B1 (ATCC Accession No. HB-12441) and the cell line designated 8G6 (ATCC Accession No. HB-12657).

9. The monoclonal antibody produced by the hybridoma cell line of claim 7.

10. The monoclonal antibody produced by the hybridoma cell line of claim 8.

11. A polypeptide useful for generating the monoclonal antibody of claim 9 or claim 10, said polypeptide consisting of the amino acid sequence AAEGLDTQRFS (SEQ ID NO:2).

12. The polypeptide of claim 11, wherein the polypeptide consists of the amino acid sequence AAEGLDTQRFSG (SEQ ID NO: 1).

13. A kit for use in isolating CD8⁺ cells which comprises, in separate compartments,
(a) an antibody which specifically binds to the sequence AAEGLDTQRFSG (SEQ ID NO:1) on CD8 molecules present on the surface of CD8+ cells, but does not activate the CD8+ cells once bound thereto; and
(b) an agent which causes the dissociation of a CD8+ cell-antibody complex, wherein the agent is the polypeptide comprising the sequence AAEGLDTQRFSG (SEQ ID NO:1).

14. A polypeptide useful for generating the monoclonal antibody of claim 9, said peptide consisting essentially of the amino acid sequence NKPKAAEGLDTQRFSGKRLG (SEQ ID NO:3).

## Patentansprüche

1. Verfahren zum Isolieren von CD8+-Zellen, das folgende Schritte umfasst:
(a) Inkontaktbringen einer Probe von isolierten mononukleären Zellen des peripheren Bluts mit einem ersten Antikörper, der spezifisch an ein Peptid, das im Wesentlichen aus AAEGLDTQRFSG (SEQ ID Nr. 1) besteht, an CD8-Moleküle bindet, die auf der Oberfläche von menschlichen CD8+-Zellen vorhanden sind, der die CD8+-Zellen jedoch nicht aktiviert, wenn er einmal daran gebunden ist; unter Bedingungen, welche die Bildung eines ersten Komplexes zwischen CD8+-Zellen und dem ersten Antikörper erlauben;
(b) Trennen der Probe von jedem ersten Antikörper, der nicht in dem ersten Komplex vorhanden ist;
(c) Inkontaktbringen der Probe mit einem immobilisierten zweiten Antikörper, der spezifisch an den ersten Antikörper in dem erstem Komplex bindet, unter Bedingungen, welche die Bildung eines immobilisierten zweiten Komplexes zwischen dem ersten Komplex und dem zweiten Antikörper erlauben, wodurch die CD8+-Zellen, die in der Probe vorhanden sind, immobilisiert werden;
(d) Trennen des immobilisierten zweiten Komplexes von der Probe;
(e) Inkontaktbringen des immobilisierten zweiten Komplexes mit einem Mittel, das ein Peptid umfasst, das AAEGLDTQRFSG (SEQ ID Nr. 1) umfasst, das unter geeigneten Bedingungen eine Dissoziation des zweiten Komplexes in CD8+-Zellen und einen immobilisierten dritten Komplex zwischen dem ersten Antikörper und dem zweiten Antikörper veranlasst; und
(f) Trennen des immobilisierten dritten Komplexes von den CD8+-Zellen, wodurch die CD8+-Zellen isoliert werden.

2. Verfahren nach Anspruch 1, wobei die CD8+-Zellen menschliche CD8+-Zellen sind.

3. Verfahren nach Anspruch 1, wobei der erste Antikörper ein monoklonaler Antikörper ist.

4. Verfahren nach Anspruch 3, wobei der monoklonale Antikörper durch eine Hybridomzelllinie produziert wird, die ausgewählt ist aus der Gruppe, bestehend aus der Zelllinie, die mit 37B1 (ATCC-Zugangsnr. HB-12441) bezeichnet ist, und der Zelllinie, die mit 8G6 (ATCC-Zugangsnr. HB-12657) bezeichnet ist.

5. Verfahren nach Anspruch 1, wobei der immobilisierte zweite Antikörper einen Antikörper umfasst, der funktionsfähig an einem magnetischen *Bead* angebracht ist.

6. Verfahren nach Anspruch 1, wobei das Mittel, welches die Dissoziation des immobilisierten dritten Komplexes veranlasst, das Polypeptid ist, das mit CD8-3 bezeichnet ist, welches die Aminosäuresequenz AAEGLDTQRFSG (SEQ ID NR. 1) umfasst.

7. Hybridomzelllinie, die einen monoklonalen Antikörper produziert, der spezifisch an ein Peptid, das im Wesentlichen aus AAEGLDTQRFSG (SEQ ID Nr. 1) besteht, an CD8-Moleküle bindet, die auf der Oberfläche von CD8+-Zellen vorhanden sind.

8. Hybridomzelllinie, die mit 37B1 (ATCC-Zugangsnr. HB-12441) bezeichnet ist, und die Zelllinie, die mit 8G6 (ATCC-Zugangsnr. HB-12657) bezeichnet ist.

9. Monoklonaler Antikörper, der durch die Hybridomzelllinie nach Anspruch 7 produziert wird.

10. Monoklonaler Antikörper, der durch die Hybridomzelllinie nach Anspruch 8 produziert wird.

11. Polypeptid, das zum Erzeugen des monoklonalen Antikörpers nach Anspruch 9 oder Anspruch 10 nützlich ist, wobei das Polypeptid aus der Aminosäuresequenz AAEGLDTQRFS (SEQ ID Nr. 2) besteht.

12. Polypeptid nach Anspruch 11, wobei das Polypeptid aus der Aminosäuresequenz AAEGLDTQRFSG (SEQ ID Nr. 1) besteht.

13. Kit zur Verwendung beim Isolieren von CD8+-Zellen, das in getrennten Fächern Folgen: des umfasst
(a) einen Antikörper, der spezifisch an die Sequenz AAEGLDTQRFSG (SEQ ID Nr. 1) an CD8-Moleküle bindet, die auf der Oberfläche von CD8+-Zellen vorhanden sind, der die CD8+-Zellen jedoch nicht aktiviert, wenn er einmal daran gebunden ist; und
(b) ein Mittel, welches die Dissoziation eines CD8+-Zell-Antikörper-Komplexes veranlasst, wobei das Mittel das Polypeptid ist, welches die Sequenz AAEGLDTQRFSG (SEQ ID Nr. 1) umfasst.

14. Polypeptid, das zum Erzeugen des monoklonalen Antikörpers nach Anspruch 9 nützlich ist, wobei das Peptid im Wesentlichen aus der Aminosäuresequenz NKPKAAEGLDTQRFSGKRLG (SEQ ID Nr. 3) besteht.

## Revendications

1. Procédé pour isoler des cellules CD8+, qui comprend les étapes consistant à :
(a) mettre en contact un échantillon de cellules mononucléaires de sang périphérique isolées avec un premier anticorps qui se fixe spécifiquement à un peptide constitué essentiellement de AAEGLDTQRFSG (SEQ ID N° 1), sur des molécules de CD8 présentes sur la surface de cellules CD8+ humaines mais qui n'active pas les cellules CD8+ une fois qu'il est fixé à celles-ci, dans des conditions permettant la formation d'un premier complexe entre les cellules CD8+ et le premier anticorps ;
(b) séparer de l'échantillon tout premier anticorps non présent dans le premier complexe ;
(c) mettre en contact l'échantillon avec un second anticorps immobilisé qui se fixe spécifiquement au premier anticorps dans le premier complexe, dans des conditions permettant la formation d'un deuxième complexe immobilisé entre le premier complexe et le second anticorps, en immobilisant ainsi les cellules CD8+ présentes dans l'échantillon ;
(d) séparer de l'échantillon le deuxième complexe immobilisé ;
(e) mettre en contact le deuxième complexe immobilisé avec un agent comprenant un peptide comprenant AAEGLDTQRFSG (SEQ ID N° 1) qui, dans des conditions convenables, provoque une dissociation du deuxième complexe en cellules CD8+ et un troisième complexe immobilisé entre le premier anticorps et le second anticorps ; et
(f) séparer des cellules CD8+ le troisième complexe immobilisé, en isolant ainsi les cellules CD8+.

2. Procédé selon la revendication 1, dans lequel les cellules CD8+ sont des cellules CD8+ humaines.

3. Procédé selon la revendication 1, dans lequel le premier anticorps est un anticorps monoclonal.

4. Procédé selon la revendication 3, dans lequel l'anticorps monoclonal est produit par une lignée cellulaire d'hybridome choisie dans le groupe constitué par la lignée cellulaire appelée 37B1 (N° d'accès ATCC HB-12441) et la lignée cellulaire appelée 8G6 (N° d'accès ATCC HB-12657).

5. Procédé selon la revendication 1, dans lequel le second anticorps immobilisé comprend un anticorps fixé de façon opérable à une bille magnétique.

6. Procédé selon la revendication 1, dans lequel l'agent qui provoque la dissociation du troisième complexe immobilisé est le polypeptide appelé CD8-3 comprenant la séquence d'acides aminés AAEGLDTQRFSG (SEQ ID N° 1).

7. Lignée cellulaire d'hybridome qui produit un anticorps monoclonal qui se fixe spécifiquement à un peptide constitué essentiellement de AAEGLDTQRFSG (SEQ ID N° 1) sur des molécules de CD8 présentes sur la surface de cellules CD8+.

8. Lignée cellulaire d'hybridome appelée 37B1 (N° d'accès ATCC HB-12441) et lignée cellulaire appelée 8G6 (N° d'accès ATCC HB-12657).

9. Anticorps monoclonal produit par la lignée cellulaire d'hybridome de la revendication 7.

10. Anticorps monoclonal produit par la lignée cellulaire d'hybridome de la revendication 8.

11. Polypeptide utile pour générer l'anticorps monoclonal de la revendication 9 ou de la revendication 10, ledit polypeptide étant constitué de la séquence d'acides aminés AAEGLDTQRFS (SEQ ID N° 2).

12. Polypeptide selon la revendication 11, lequel polypeptide est constitué de la séquence d'acides aminés AAEGLDTQRFSG (SEQ ID N° 1).

13. Trousse à utiliser pour isoler des cellules CD8+, qui comprend, dans des compartiments séparés :
(a) un anticorps qui se fixe spécifiquement à la séquence AAEGLDTQRFSG (SEQ ID N° 1) sur des molécules de CD8 présentes sur la surface de cellules CD8+, mais n'active pas les cellules CD8+ une fois qu'il est fixé à celles-ci ; et
(b) un agent qui provoque la dissociation d'un complexe de cellule CD8+/anticorps, lequel agent est le polypeptide comprenant la séquence AAEGLDTQRFSG (SEQ ID N° 1).

14. Polypeptide utile pour générer l'anticorps monoclonal de la revendication 9, ledit peptide étant constitué essentiellement de la séquence d'acides aminés NKPKAAEGLDTQRFSGKRLG (SEQ ID N° 3).
